# EUROPEAN PATENT SPECIFICATION

(11) **EP 1 224 464 B1**
(45) Date of publication and mention of the grant of the patent: **09.06.2004**
(21) Application number: 00971595.4
(22) Date of filing: 27.10.2000
(51) Int. Cl.: G01N 33/558, G01N 33/543

(54) **LATERAL FLOW DEVICE UTILISING PARTICULATE CARRIER**
LATERALE DURCHFLUSSVORRICHTUNG MIT TEILCHENFÖRMIGEM TRÄGER
DISPOSITIF A ECOULEMENT LATERAL FAISANT APPEL A UN SUPPORT DE PARTICULES

(30) Priority: 27.10.1999 GB 9925461
(43) Date of publication of application: 24.07.2002
(73) Proprietor: Genosis Limited, Kingston-upon-Thames KT2 5AA (GB)
(72) Inventor: FORREST, Gordon, Coulter, Genosis Limited, Kingston-upon-Thames KT2 5AA (GB)
(74) Representative: Fisher, Adrian John
(86) International application number: PCT/GB2000/004140
(87) International publication number: WO 2001/031337

(56) References cited:
- EP-A- 0 297 292
- WO-A-00/20866
- WO-A-87/03690
- US-A- 5 770 460

## Description

### TECHNICAL FIELD

This invention is in the field of assay devices. More specifically, it relates to an improved lateral flow assay device, utilising size-based particle capture.

### BACKGROUND ART

The format of the standard rapid test lateral flow device has remained unchanged for around ten years. Typically, the device will comprise a nitrocellulose strip. Sample is applied to an application zone, from which it flows by capillary action through a zone containing a visibly-labelled antibody specific for the analyte in question. Free and bound label continue to migrate to a capture zone, where immobilised antibody specific for the analyte binds the analyte-label complex. Free label (unbound antibody) continues to migrate, leaving an analyte-specific signal in the capture zone.

These types of lateral flow device are disclosed in, for example, EP-A-0284232, but are unsatisfactory in many respects.

Firstly, manufacturing quality control is difficult. The solid phase capture membrane is typically made from nitrocellulose, and antibodies are applied to the membrane directly. Nitrocellulose manufacture is not, however, homogeneous. Quality control of the solid phase antibody is therefore limited to testing a statistical sample of devices from the same, but heterogeneous, batch, and assuming that the whole batch will perform within specific tolerances. It is well known, however, that membranes vary considerably, even within a single batch or lot number.

Secondly, they are relatively cumbersome to manufacture. The application of immobilised antibody to the strip requires a separate step from the application of the mobile labelled antibody. The capture antibody can be sprayed directly onto the nitrocellulose strip, but the label antibody has to be soaked into material which is subsequently attached to the nitrocellulose strip, with an overlap to ensure capillary flow.

Thirdly, antibody is immobilised by spraying a solution onto a membrane. Some of the antibody does not bind to the membrane strongly, however, and some remains loosely associated with immobilised antibody. This semi-bound or unbound antibody can become mobile when the solvent front passes over it, resulting in lower binding of label at the detection zone. If the device includes a control line, this will capture the additional label which should have been captured at the detection zone. Tests that rely on a comparison of colour intensity between control and detection lines, such as ovulation prediction kits, may therefore give false results. Furthermore, application by spraying inevitably leads to diffusion into the membrane, leading to a more diffuse and less focused detection signal.

Fourthly, the sensitivity of the devices is limited by their format. Analyte and labelled-antibody react as they migrate through the membrane, and flow rates are therefore adjusted to enable the labelled-antibody to flow at the solvent front in order to maximise the amount of time in which the analyte-label complex can form. The complex passes over the capture antibody for a short time, however, thus imposing constraints on the design of the test and its performance characteristics. The short reaction time decreases sensitivity, and also means that high affinity capture antibodies are required.

Finally, the shelf-life of these test devices is often limited by the collapse of the immobilised capture antibody onto the membrane over time.

These shortcomings in the prior art devices are addressed by the present invention, which does not use immobilised antibody to capture an analyte-label complex.

### DISCLOSURE OF THE INVENTION

The invention provides a lateral flow device for assaying an analyte, having a porous reaction zone in communication with a porous filter zone, wherein the reaction zone contains (i) an analyte-specific label and (ii) a particulate carrier having an analyte-specific capture reagent immobilised thereon, and wherein the filter zone has a smaller pore size than that of the reaction zone, such that label that is not bound to the particulate carrier can migrate into the filter zone, whereas label that is bound to the particulate carrier cannot.

After a sample is applied to the device, lateral flow through the device brings analyte in the sample into contact with label and particulate carrier within the reaction zone, where it can form a label-analyte-particulate carrier complex. Free label does not bind to the particulate carrier. During flow from the reaction zone to the filter zone, free label can migrate into and through the filter zone, whereas the particle-analyte-label complex is too large, and is captured at the entrance to the filter zone. As analyte concentration increases, the amount of label retained at the entrance also increases, thus allowing quantitative measurement of analyte.

Rather than use immobilised antibody to capture an analyte-label complex, therefore, the invention captures the complex on a large particulate carrier, which is then prevented from further lateral flow by being 'filtered' when the pore size of the device decreases. The capture and label reagents can thus be applied to the device without the need for separate spraying and adhesion steps. Furthermore, in contrast to typical prior art devices, the label and the capture reagent both have ample time to interact with the antigen as they co-migrate, giving increased sensitivity.

The reaction zone is where analyte, analyte-specific label and particulate carrier come into contact. It is preferably formed from fibrous material, such as a pad of HDPE material, glass fibre, or the like with a pore size larger than the particulate carrier. In an alternative configuration, the reaction zone may comprise appropriately-sized channels or grooves along which capillary flow can take place.

It will be appreciated that the reaction zone may be divided into sub-zones containing different reagents. For example, the reagents might be arranged such that analyte migrating therethrough first meets label and subsequently meets particulate carrier; alternatively, they might be arranged such that carrier is encountered before label, or such that label and carrier are encountered simultaneously. The precise arrangement of reagents in the reaction zone is thus not important, as long as the label, analyte and particulate carrier are able to interact in order to form complexes, before flowing into the filter zone.

The device may have a sample application zone separate from the reaction zone, upstream or downstream. In an alternative arrangement, the sample may be applied directly to the reaction zone.

The label is any reagent which can detectably and specifically bind to the analyte. It is typically an antibody which can bind to the analyte, and which has been suitably tagged. The label is preferably visible to the naked eye *eg*. a fluorescent label, or a particulate label such as colloidal gold (which is visible as a pink colour), or a stain such as eosin. It will be appreciated that the term 'antibody' may include polyclonal and monoclonal antibodies, as well as antibody fragments (*eg*. F(ab)₂, Fc *etc*.) or derivatives, provided that the necessary biological specificity is retained. Monoclonal antibodies are preferred.

It will be apparent that the invention relies upon the label being smaller than the particulate carrier, such that the flow of free label is not retarded by the filter zone.

The particulate carrier may be anything that can bind to an analyte-label complex and which is of a size such that it can migrate through the pores in the reaction zone, but cannot migrate through the pores in the filter zone. It may be based on mono-dispersed particles such as beads, liposomes, microparticles, microspheres, aggregates, polymeric antibodies *etc*. Preferably, it is a polymeric bead or particle, such as a latex bead. The surface of the particle will typically be coated with immobilised capture reagent, specific for analyte-label complex. The capture reagent is preferably an antibody which can bind to the analyte of interest, at a different epitope from the label. As mentioned above, the term 'antibody' may take various forms, provided that the necessary biological specificity is retained, but monoclonal antibodies are preferred.

In contrast to capture reagent that are bound directly onto the test device membrane, particle-immobilised reagent can undergo washing, testing, reagent processing quality control *etc.* before being applied to the device. In the prior art devices, capture antibody cannot undergo reagent processing, with associated problems for quality control. Furthermore, there is no binding interaction between the substrate of the device and the analyte. The device becomes a simple fluid conduit, rather than being directly involved in the assay via antibody immobilised thereon.

The filter zone can be made from any suitable porous material through which free label can migrate, whilst label-analyte-particulate carrier complexes cannot. This requirement is reflected in the pore size of the filter zone, which is smaller than that of the reaction zone. Typically, the filter zone will be made from nitrocellulose.

Suitable pore sizes for the labelling and filter zones are evidently related to the size of the particulate carrier. The filter zone will typically have a nominal pore size of around 1-15µm, preferably 3-10µm, and more preferably 5-8µm. A preferred size for the particulate carrier is a diameter of 3µm, but the invention can operate with a wide variety of pore and particle sizes. Reaction zone pore size, capture zone pore size and particulate carrier size can also be selected to achieve a particular lateral flow rate through the device.

In some embodiments, the filter zone and reaction zone may be formed from a single piece of porous material, which contains a region of reduced pore size. By crushing or compressing a region of a porous material such as nitrocellulose, for instance, its pore size can be reduced such that a particle-analyte-label complex cannot enter the compressed region *ie.* to form a filter zone. As an alternative, the pores of the material could be partially blocked, to achieve the same effect.

As is well known to those in the art, the nominal pore size of a porous material can be determined by hard particle challenge testing *ie*. by determining the maximum diameter of spherical particles which can pass through the material. Alternatively, the pore size of a fibrous material may be determined by measuring its 'bubble point'. The bubble point is the pressure required to force air through a (water) wet membrane, and correlates with the pore size as measured by particle retention (although at extremes of pressure and pore size, the correlation may be weaker). Measurement of the flow rate through a material can also be used to determine pore size.

The entrance to the filter zone is preferably narrow in comparison to the total length of the filter zone, so that the retained particle-analyte-label complex is focused to give a sharp signal. Where the reaction zone and the filter zone are formed from strips of overlapping material, the overlap should thus be narrow. This can be achieved, for instance, by the presence of a non-porous material covering the majority of the overlap.

The analyte is any material capable of forming a specific binding interaction with a complementary material. It is preferably a hormone, such as follicle stimulating hormone (FSH), human chorionic gonadotrophin (hCG) or luteinizing hormone (LH).

In preferred embodiments, migration of a sample to the filter zone is assisted by upstream and downstream wicks to aid capillary movement.

The device of the invention can be produced simply and cheaply, conveniently in the form of a test strip or dipstick. Furthermore, it can be used very easily, for instance by the home user. The invention thus provides an assay device which can be used at home as a basic screen of, for instance, female fertility or pregnancy.

The invention also provides a similar device to that described above, adapted for a competitive assay format, rather than a sandwich format. The invention thus provides a lateral flow device for assaying an analyte, having a porous reaction zone in communication with a porous filter zone, wherein the reaction zone contains (i) labelled analyte and (ii) a particulate carrier having an analyte-specific capture reagent immobilised thereon, and wherein the filter zone has a smaller pore size than that of the reaction zone, such that label that is not bound to the particulate carrier can migrate into the filter zone, whereas label that is bound to the particulate carrier cannot.

In the competitive format, lateral capillary flow through the device after a sample is applied brings analyte in the sample into contact with the labelled analyte and the particulate carrier within the reaction zone. The labelled analyte and the sample analyte compete for analyte-specific reagent immobilised on the particulate carrier. During flow from the reaction zone to the filter zone, the particulate carrier, in association with either the sample analyte or the labelled analyte, is captured at the entrance to the filter zone. As analyte concentration in the sample increases, the amount of label retained at the entrance decreases, thus allowing quantitative measurement of analyte. In the competitive format, the device will typically also comprise a downstream zone for capturing free label, to allow a comparison with the signal at the entrance to the filter zone.

It will be appreciated that, in the competitive format, the labelled analyte in the reaction zone may alternatively be a labelled analogue of the analyte, provided that the ability to bind the capture reagent is retained.

### BRIEF DESCRIPTION OF DRAWINGS

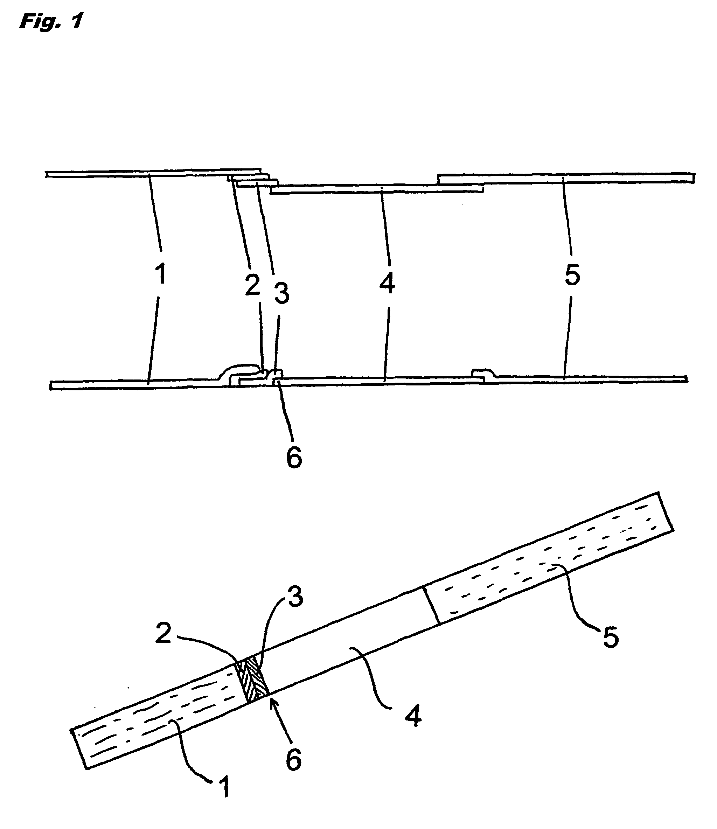

Figure 1 shows a device according to the invention.

### MODES FOR CARRYING OUT THE INVENTION

### Coating of Latex Particles with Antibody

Polystyrene particles (Sigma, LB30), 3µm diameter, were diluted from 10% to 1% solids in borate buffer (pH8.5, 10mM). The particles were washed by centrifugation at 3000rpm for 5 minutes and the supernatant was replaced by an equal volume of fresh borate buffer.

Monoclonal anti-hCG at 5mg/ml was added to the latex particles to give a final concentration of 150µg/ml. The suspension was mixed for 1 hour at 20°C before the addition of BSA to a concentration of 0.02% (w/v). The suspension was mixed for a further 30 minutes at 20°C.

The suspension was centrifuged at 3000rpm for 5 minutes and the supernatant discarded. The pellet was re-suspended in borate buffer (pH 8.5, 10mM) to give a concentration of 1%(w/v), then washed twice, and each time re-suspended to 1% solids in borate buffer (pH8.5, 10mM).

### Construction of device

The device shown in Figure 1 comprises a lower wick (1), a first absorbent pad (2), a second absorbent pad (3), a nitrocellulose membrane (4), and an upper wick (5).

The lower wick material (1) is formed from Ahlstrom blotting grade paper (code 222). The paper was saturated with a solution of 10mM Tris containing 1% Tween-20, pH 8.2, and was dried at 70 °C.

The first absorbent pad (2), was formed by saturating Ahlstrom glass fibre material (Code 8980), with a solution containing a second monoclonal anti-hCG antibody (which recognises a different epitope from the latex-conjugated antibody), conjugated to 40nm gold, and diluted in 5% trehalose. Pad (2) was dried at room temperature and stored in a sealed pouch with desiccant prior to use.

The second absorbent pad (3) was formed by saturating Sintair material (100µm pore size pre-treated with 1% BSA and 1% Triton) with the conjugated latex particles, pre-diluted to 0.2% solids in trehalose 5% (w/v). Pad (3) was dried at room temperature and stored in a sealed pouch with desiccant prior to use.

The nitrocellulose membrane (4) (Millipore SHNF04000) is 25x5mm wide is mounted on a plastic card for support.

Upper wick (5) is formed from untreated Whatman chromtography paper 3MM CHR cat no 3030640, 30mm wide) and overlaps the nitrocellulose membrane by 5mm.

The components were assembled onto the card-mounted nitrocellulose membrane (4). The second absorbent pad (3), 5mm x 5mm, was attached to the nitrocellulose (4) with a 1mm overlap. Overlapping the second absorbent pad (3) by 4mm is the first absorbent pad (2). The first absorbent pad is covered by the lower wick material (1), leaving 1mm of pad visible.

The overall dipstick is 5mm wide and 7.5cm long.

It will be appreciated that an alternative sequence of reagent-analyte contact can be arranged by assembling the components in a different order *(e.g.* so that sample applied to lower wick (1) encounters the latex particles before it encounters the gold conjugate during the lateral flow from lower wick (1) to upper wick (5)).

### Assay utilising the device

To use the device, a suitable biological sample (*e.g*. urine) is applied to the lower wick (1). A volume of around 160µl is typically sufficient for the device described above. The sample migrates through the test strip, mobilising in turn the gold and latex conjugates. In the presence of hCG analyte, a complex is formed between the Ab-latex-analyte-gold-Ab which, because of its size, becomes trapped at the interface (6) with the nitrocellulose membrane. The presence of the gold produces a clear, discrete red signal at interface (6), the intensity of which is dependent on the amount of hCG present in the sample. In the absence of hCG, the gold migrates up the nitrocellulose, but latex particles are still retained at interface (6). No signal is visible at interface (6) because of the white colour of the latex beads.

### Example

160µl of PBS containing 100IU/L of hCG was placed in microtitre well A.

160µl of PBS was placed in microtitre well B.

A device was placed in each well, and the contents of the wells were allowed to absorb into the wick and migrate through the membrane by capillary action.

After ten minutes, a clear red line was visible at interface (6) of the device in well A. The device in well B showed no such signal.

## Claims

1. A lateral flow device for assaying an analyte, having a porous reaction zone in communication with a porous filter zone, wherein the reaction zone contains (i) an analyte-specific label and (ii) a particulate carrier having an analyte-specific capture reagent immobilised thereon, and wherein the filter zone has a smaller pore size than that of the reaction zone, such that label that is not bound to the particulate carrier can migrate into the filter zone, whereas label that is bound to the particulate carrier cannot.

2. A lateral flow device for assaying an analyte, having a porous reaction zone in communication with a porous filter zone, wherein the reaction zone contains (i) labelled analyte, or analogue thereof, and (ii) a particulate carrier having an analyte-specific capture reagent immobilised thereon, and wherein the filter zone has a smaller pore size than that of the reaction zone, such that label that is not bound to the particulate carrier can migrate into the filter zone, whereas label that is bound to the particulate carrier cannot.

3. The device of claim 1 or claim 2, wherein the label is an antibody which can bind to the analyte, and which carries a visible tag.

4. The device of claim 3, wherein the visible tag is colloidal gold

5. The device of any preceding claim, wherein the particulate carrier is a latex bead coated with an immobilised antibody specific for the analyte.

6. The device of any preceding claim, wherein the entrance to the filter zone is narrow in comparison to the total length of the filter zone

7. The device of any preceding claim for assaying a hormone.

8. The device of any preceding claim for sandwich or competitive assay.

## Patentansprüche

1. Laterale Durchflussvorrichtung zur Analyse eines Analyts mit einer porösen Reaktionszone in Verbindung mit einer porösen Filterzone, wobei die Reaktionszone
(i) einen Analyt-spezifischen Indikator und
(ii) einen partikelförmigen Träger mit einem darauf mobilisierten Analyt-spezifischen Aufnahmereagens enthält und
wobei die Filterzone eine kleinere Porengröße als die der Reaktionszone aufweist, so dass der Indikator, der nicht an den partikelförmigen Träger gebunden ist, in die Filterzone migrieren kann, wohingegen Indikator, der an dem partikelförmigen Träger gebunden ist, nicht in die Filterzone migrieren kann.

2. Durchflussvorrichtung zur Analyse eines Analyts mit einer porösen Reaktionszone in Verbindung mit einer porösen Filterzone, wobei die Reaktionszone
(i) ein markiertes Analyt oder ein Analog davon und
(ii) einen partikelförmigen Träger mit einem darauf mobilisierten Analyt-spezifischen Aufnahmereagens enthält.

3. Vorrichtung nach Anspruch 1 oder 2, wobei der Indikator ein Antikörper ist, der an dem Analyt gebunden werden kann und der eine sichtbare Markierung trägt.

4. Vorrichtung nach Anspruch 3, wobei die sichtbare Markierung kolloidales Gold ist.

5. Vorrichtung nach einem der vorhergehenden Ansprüche, wobei der partikelförmige Träger eine Latexleiste ist, die mit einem immobilisierten Antikörper ummantelt ist, der spezifisch für das Analyt ist.

6. Vorrichtung nach einem der vorhergehenden Ansprüche, wobei der Eingang in die Filterzone eng im Vergleich zur Gesamtlänge der Filterzone ist.

7. Vorrichtung nach einem der vorhergehenden Ansprüche zum Analysieren eines Hormons.

8. Vorrichtung nach einem der vorhergehenden Ansprüche zur Sandwich-Analyse oder zur Konkurrenz-Analyse.

## Revendications

1. Dispositif à écoulement latéral destiné à doser un analyte, comportant une zone de réaction poreuse en communication avec une zone de filtrage poreuse, dans lequel la zone de réaction contient (i) un marqueur spécifique à l'analyte, et (ii) un support particulaire sur lequel est immobilisé un réactif de capture spécifique à l'analyte, et dans lequel la zone de filtrage présente une taille de pore plus faible que celle de la zone de réaction, de telle sorte que du marqueur qui n'est pas lié au support particulaire peut migrer dans la zone de filtrage, alors que du marqueur qui est lié au support particulaire ne le peut pas.

2. Dispositif à écoulement latéral destiné à doser un analyte, comportant une zone de réaction poreuse en communication avec une zone de filtrage poreuse, dans lequel la zone de réaction contient (i) un analyte marqué ou analogue de celui-ci, et (ii) un support particulaire sur lequel est immobilisé un réactif de capture spécifique à l'analyte, et dans lequel la zone de filtrage présente une taille de pore plus faible que celle de la zone de réaction, de telle sorte que du marqueur qui n'est pas lié au support particulaire peut migrer dans la zone de filtrage, alors que du marqueur qui est lié au support particulaire ne le peut pas.

3. Dispositif selon la revendication 1 ou la revendication 2, dans lequel le marqueur est un anticorps qui peut être lié à l'analyte et qui porte une étiquette visible.

4. Dispositif selon la revendication 3, dans lequel l'étiquette visible est de l'or colloïdal.

5. Dispositif selon l'une quelconque des revendications précédentes, dans lequel le support particulaire est une perle de latex revêtue d'un anticorps immobilisé spécifique à l'analyte.

6. Dispositif selon l'une quelconque des revendications précédentes, dans lequel l'entrée de la zone de filtrage est étroite par comparaison avec la longueur totale de la zone de filtrage.

7. Dispositif selon l'une quelconque des revendications précédentes, destiné à doser une hormone.

8. Dispositif selon l'une quelconque des revendications précédentes, destiné à un dosage intercalé ou comparatif.
